(19) Europäisches Patentamt
European Patent Office
Office européen des brevets

(11) Publication number: **0 131 973**
**B1**

(12) # EUROPEAN PATENT SPECIFICATION

(45) Date of publication of patent specification: **02.08.89**

(21) Application number: **84200659.5**

(22) Date of filing: **08.05.84**

(51) Int. Cl.[4]: **C 07 D 233/64,**
C 07 D 233/70,
C 07 D 233/88,
C 07 D 233/84,
C 07 D 233/68, A 61 K 31/415

(54) **New amidine derivatives of 2-substituted 4-phenilimidazole.**

(30) Priority: **18.07.83 IT 2211083**

(43) Date of publication of application:
**23.01.85 Bulletin 85/04**

(45) Publication of the grant of the patent:
**02.08.89 Bulletin 89/31**

(84) Designated Contracting States:
**AT BE CH DE FR IT LI LU NL SE**

(56) References cited:
**EP-A-0 053 407**

(73) Proprietor: **ISTITUTO DE ANGELI S.p.A.**
**Via Serio 15**
**I-20139 Milano (IT)**

(72) Inventor: **Bietti, Giuseppe**
**Via Lario 31**
**I-20159 Milan (IT)**
Inventor: **Cereda, Enzo**
**Via Romagnolo 2A**
**I-15057 Tortona, Alessandria (IT)**
Inventor: **Donetti, Arturo**
**Viale Romagna 4**
**I-20133 Milan (IT)**
Inventor: **Giachetti, Antonio**
**Via Guerrini 3**
**I-20133 Milan (IT)**
Inventor: **Pagani, Ferdinando**
**Via Morigiola 18**
**I-2005 Overano Brianza, Milan (IT)**

(74) Representative: **Appoloni, Romano et al**
**ING. BARZANO' & ZANARDO MILANO S.p.A. Via Borgonuovo 10**
**I-20121 Milano (IT)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 131 973 B1

Courier Press, Leamington Spa, England.

## Description

This invention relates to novel amidine derivatives of 2-substituted 4-phenylimidazole, to the processes for their preparation, and to the pharmaceutical compositions containing them. The new compounds are $H_2$-receptor blocking agents and may be used as gastric acid secretion inhibitors and antiulcer agents.

EP—A—53 407 discloses a group of imidazolylphenyl formamidines which show an $H_2$-receptor blocking effect.

According to the present invention there are provided amidine derivatives of 2-substituted 4-phenylimidazole of general formula I

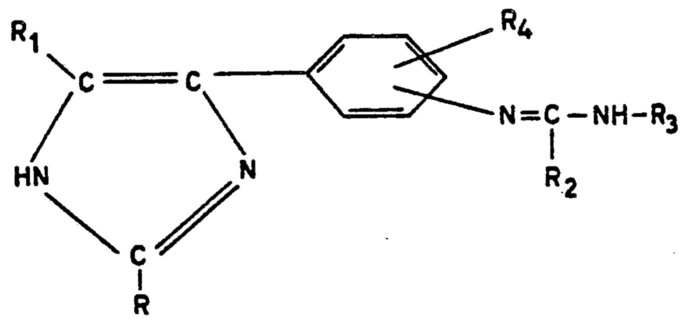
(I)

in which R represents a linear or branched $C_{1-4}$ alkyl group, a hydroxy group, a $C_{1-3}$ alkoxy group, a mercapto, a $C_{1-3}$ alkylthio group, a halogen atom, a $C_{1-3}$ alkylsulfinyl or $C_{1-3}$ alkylsulphonyl group, a sulfamoyl group, an amino group which may be substituted by one or two alkyl groups having 1 to 3 carbon atoms, a $C_{1-4}$ alkanoyl-amino or phenyl groups; $R_1$ and $R_2$ which may be the same or different, represent a hydrogen atom or a $C_{1-4}$ alkyl group; $R_3$ represents a linear or branched $C_{1-8}$ alkyl, hydroxy-$(C_{1-6})$alkyl, $(C_{1-6})$alkoxy-$(C_{1-6})$alkyl, $(C_{1-6})$alkylthio-$(C_{1-6})$alkyl, cyano$(C_{1-6})$alkyl, a $C_{3-5}$ alkenyl group, an alkynyl group having 3 to 4 carbon atoms, a cyano group, an aryl group optionally substituted by halogen, a benzyl group, a $(C_{3-6})$cycloalkyl group, a $(C_{3-6})$cycloalkyl$(C_{1-2})$alkyl group, an aromatic 5- to 6-membered heterocyclic ring containing one hetero atom; $R_4$ represents a hydrogen atom, a $C_{1-3}$alkyl or $C_{1-3}$alkoxy group, a halogen atom, a cyano or carbamoyl group, tautomeric forms thereof and acid addition salts thereof.

For pharmaceutical use the acid addition salts referred to the above will be physiologically compatible acid addition salts. The term "acid addition salts" includes salts with inorganic or organic acids. Suitable physiologically compatible acid addition salts include, for example, salts formed with acetic, maleic, fumaric, citric, hydrochloric, tartaric, hydrobromic, nitric or sulphuric acid.

It is understood that for convenience in this specification reference will be made indifferently either to the compounds as bases or to the corresponding salts.

It is to be pointed out that, although in the formula (1) the double bond in the amidine radical and in the imidazolyl ring has been inserted in a particular position, other different tautomeric forms are possible. Furthermore, the compounds in which R represents a hydroxy or mercapto group, may be in the corresponding imidazolinone or imidazolinthione forms. The present invention includes such tautomeric forms as regards both the compounds and their processes of preparation.

In the above mentioned formula (I) the amidine radical may be in the ortho, meta or para position of the benzene ring with respect to the imidazolyl group, and $R_4$ in any position in the benzene ring.

The compounds of general formula (I) may, for example, be prepared by the following processes which constitute further features of the invention:

Process A. Reaction of a compound of general formula II

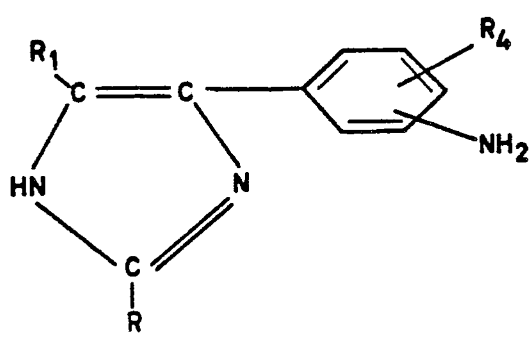
(II)

(wherein R, $R_1$ and $R_4$ are as hereinbefore defined) with a reactive derivative of a carboxamide of formula III

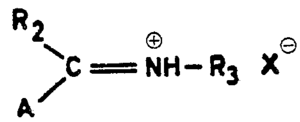

(III)

where $R_2$ and $R_3$ are as hereinbefore defined, $X^{\ominus}$ represents the anion of an inorganic acid such as hydrochloride or fluoroborate, and A represents a benzoyloxy group, chlorine or a lower alkoxy group such as methoxy or ethoxy. If desired the compound of formula III may be made to react also as a base. The reaction is generally effected at a temperature from 0 to 100°C preferably from 20 to 60°C. The reaction is advantageously carried out in the presence of an inert organic solvent. Suitable solvents include for example alcohols having 1 to 3 carbon atoms such as methanol or ethanol, halogenated hydrocarbons such as dichloromethane, dioxane or acetone.

The compounds of formula III used as starting material in the above process may be obtained by conventional methods by reacting a carboxamide of the formula IV

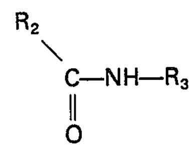

(IV)

wherein $R_2$ and $R_3$ are as above defined, with benzoylchloride, triethyloxomium fluoroborate, ethyl chloroformate, phosphorus oxychloride or phosphorus pentachloride.

Process B. Reaction of a N,N'-disubstituted amidine compound of formula V

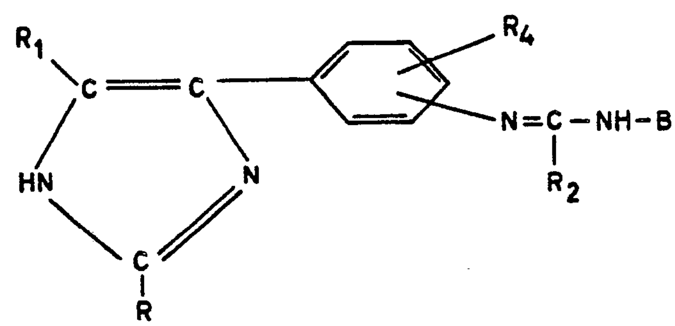

(V)

wherein R, $R_1$, $R_2$ and $R_4$ are as hereinbefore defined, B represents a suitable removable group, such as a cyano, acetyl, ethoxycarbonyl or carbamyl group with an amine of formula (VI)

$$H_2N—R_3 \qquad (VI)$$

where $R_3$ is as hereinbefore defined.

The reaction is conveniently performed in the presence of water or of an inert organic solvent for example alcohols such as methanol or ethanol, formamide, dioxane or acetonitrile. This reaction may for example be effected at a temperature from 10—50°C preferably at room temperature.

The compounds of the formula (V) used as starting material in the above process are obtained by processes that are known per se in the literature, for example, by reacting a compound of formula II with a N-substituted ethyl imidate of formula VII

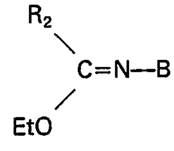

(VII)

wherein $R_2$ and B are as hereinbefore defined, or optionally, when in the compound of formula (V) B represents a cyano group, the reaction may also be carried out in a single step by reacting a compound of formula II with cyanamide in the presence of a compound of formula VIII

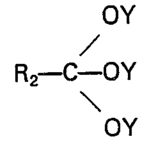

(VIII)

in which $R_2$ is as above defined and Y represents a lower alkyl group, such as methyl or ethyl.

The reaction is generally carried out in the presence of a suitable inert organic solvent for example a lower alcohol, ethers, ethylacetate, acetonitrile or dioxane or without solvent at a temperature from 20 to 80°C. The compound of formula (VII) may be prepared by conventional methods.

The compounds of general formula (I) prepared according to the processes A and B may optionally be converted with inorganic or organic acids into the corresponding physiologically compatible acid addition salts, for example, by conventional methods such as by reacting the compounds as bases with a solution of the corresponding acid in a suitable solvent. Particularly preferred acids include for example hydrochloric, sulphuric, maleic and fumaric acid. The compounds of general formula (I) and their physiologically compatible acid addition salts are $H_2$-receptor blocking agents which inhibit the gastric acid secretion.

Representative examples of the preferred compounds, according to the present invention, for their better activity as antisecretory-antiulcer agents and for the teatment of disorders of the gastrointestinal tract are:

| | |
|---|---|
| N-Ethyl-N'-[4-(2-methyl-imidazol-4-yl)phenyl]formamidine | (Compound 2) |
| N-Isopropyl-N'-[4-(2-methyl-imidazol-4-yl)phenyl]formamidine | (Compound 3) |
| N-Allyl-N'-[4-(2-methyl-imidazol-4-yl)phenyl]formamidine | (Compound 4) |
| N-t.Butyl-N'-[4-(2-methyl-imidazol-4-yl)phenyl]formamidine | (Compound 39) |
| N-Isopropyl-N'-[4-(2-hydroxy-imidazol-4-yl)phenyl]formamidine | (Compound 28) |

The antagonistic activity of compounds according to the invention on histamine $H_2$-receptors is demonstrated either "in vitro" or "in vivo" by their inhibition of the $H_2$-dependent biological effects, which include the histamine evoked positive chronotropic effect and the histamine induced gastric secretion of acid respectively.

The inhibition of the positive chronotropic effect has been investigated in isolated guinea pig atria suspended in an organ bath (50 ml) containing oxygenated ($O_2$:95%—$CO_2$:5%) Krebs-Henseleit solution (pH 7.4) maintained at 32°C. The myocardial preparation, loaded with 1 g isometric tension, is allowed to stabilize 60 min., and myocardial contractions are recorded through an isometric lever connected to a strain-gauge coupler, and the instantaneous rate is monitored with a cardiotachometer, and a heatwriting pen recorder. After two control responses to histamine ($10^{-6}$ g.ml$^{-1}$) the test compounds are added to the bath at the desired final concentration and left for 30 minutes before the atria are again challenged with histamine. The chronotropic response obtained in the presence of the antagonist is then compared to the control response to histamine and the percent reduction of the histamine $H_2$-evoked response is calculated. The average effective concentration ($EC_{50}$) of the $H_2$ antagonists is also calculated by standard procedure according to Dr. Waud, Analysis of dose-response curves, in "Methods in Pharmacology" vol. 3 Smooth muscle, Ed. Daniel E. E. Paton, M., Plenum Press, New York (1975); Ash and Schild, Br. J. Pharmacol. Chemother. *27,* 427—439, 1966. The following table shows the obtained results:

TABLE 1

"In vitro" inhibitory activity in histamine induced tachycardia (guinea pig atria)

| Compound | $EC_{50}10^{-7}$ M |
|---|---|
| 2 | 1.5 |
| 3 | 1.8 |
| 4 | 1.2 |
| 39 | 4.0 |
| 28 | 1.94 |
| CIMETIDINE | 34.0 |

The ability of the test compounds to inhibit histamine induced gastric secretion of acid, has been investigated after intravenous or intraduodenal administration in stomach perfused rats, according to Gosh and Schild (Br. J. Pharmacol. Chemother. *13,* 54, 1958).

The preparation of the animals in general anaesthesia (urethane, 1 g.kg$^{-1}$ i.p.) and constant temperature, is achieved by inserting and tying in place polyethyl tubes (PE 50) in the oesophagus and in the pyloric-antral region. After the stomach is washed to remove residuals of food, continuous perfusion of the stomach was started with saline, 0.5 ml.min$^{-1}$ (37°C), primed by a Jobling peristaltic pump. After 30 min. of perfusion adaptation, the stomach perfusate is collected in 30 min. samples, and titrated for acid content, expressed as µEq of NaOH 1 N. As control acid output becomes constant intravenous perfusion of

histamine (1 $mg.kg^{-1}hr^{-1}$) is started and maintained throughout the experimental period. After the acid secretion has reached the steadily higher level, increasing doses of the test compounds are injected intravenously in order to obtain dose response functions. $ED_{50}$ were then calculated by standard procedure.

The results are reported in the following table:

TABLE II

"In vivo" antisecretory activity in histamine induced gastric secretion (stomach perfused rat)

| Compound | $ED_{50}$ $mg.kg^{-1}$ (i.v.)* |
|---|---|
| 2 | 0.032 |
| 3 | 0.067 |
| 4 | 0.025 |
| 39 | 0.080 |
| 28 | 0.065 |
| CIMETIDINE | 0.560 |

*The values of activity are expressed taking the compound as a base.

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral or parenteral administration. Preferred forms include for example capsules, tablets, coated tablets, ampoules or vials containing lyophilized mass of the salt.

The active ingredient may be incorporated in excipients or carriers conventionally used in pharmaceutical compositions such as for example talc, gum arabic, lactose, gelatine, magnesium stearate, corn starch, aqueous or non-aqueous vehicles, polyvinylpirrolidone, mannitol.

The compositions are advantageously formulated as dosage units, each dosage unit being adapted to supply a fixed dose of the active ingredient. Each dosage unit may conveniently contain from 10 mg to 500 mg and preferably from 20 mg to 150 mg.

The following examples illustrate some of the new compounds according to the present invention; these examples are not to be in any way considered limitative of the scope of the invention itself:

Example 1

2-Methyl-4-(4-nitro-phenyl)-1H-imidazole

A mixture of 4-nitro-phenacyl bromide (100 g), acetamide (300 g), and water (15 g) was stirred at 140°C for 8 hours, then poured into water (1500 ml). The solution was filtered and made alkaline with 10% sodium hydroxide. The solid which separated was filtered, washed with water and dried to give 48.1 g of the title compound. M.p. 216—219°C.

Following the above procedure, using the appropriate phenacyl bromide and the appropriate amide as starting materials, the following nitrophenyl imidazole derivatives were prepared:

| | |
|---|---|
| 2-Methyl-4-(3-nitro-phenyl)-1H-imidazole | M.p. 146—147°C |
| 2-Ethyl-4-(4-nitro-phenyl)-1H-imidazole | M.p. 204—205°C |
| 2-Isopropyl-4-(4-nitro-phenyl)-1H-imidazole | M.p. 201—205°C |
| 2,5-Dimethyl-4-(4-nitro-phenyl)-1H-imidazole | M.p. 234—235°C |
| 2-Methyl-4-(3-bromo-4-acetamido-phenyl)-1H-imidazole | M.p. 152—154°C |
| 2-Phenyl-4-(4-nitro-phenyl)-1H-imidazole | M.p. 231—233°C |

Example 2

2-Methyl-4-(4-amino-phenyl)-1H-imidazole

2-Methyl-4-(4-nitro-phenyl)-1H-imidazole (28 g) dissolved in methanol (210 ml) was hydrogenated in the presence of Pd/C 10% (1.35 g) at atmospheric pressure and at room temperature. When the calculated amount of hydrogen was taken up, the catalyst was filtered off and the solution was evaporated to dryness

to give 23.2 g of the title compound as spongy solid. Following the above procedure, using the appropriate nitro-phenyl derivative, the following aminophenyl derivatives were prepared:

| | |
|---|---|
| 2-Methyl-4-(3-amino-phenyl)-1H-imidazole | M.p. 135—136°C |
| 2-Ethyl-4-(4-amino-phenyl)-1H-imidazole | M.p. spongy solid |
| 2-Isopropyl-4-(4-amino-phenyl)-1H-imidazole | M.p. spongy solid |
| 2,5-Dimethyl-4-(4-amino-phenyl)-1H-imidazole | M.p. 190—192°C |
| 2-Methoxy-4-(4-amino-phenyl)-1H-imidazole | M.p. 242—244°C |
| 2-Acetamido-4-(4-amino-phenyl)-1H-imidazole | M.p. 215—216°C |
| 2-Amino-4-(4-amino-phenyl)-1H-imidazole | M.p. 143—144°C |
| 2-Methyl-4-(3-methyl-4-amino-phenyl)-1H-imidazole | M.p. 135—136°C |
| 2-Methyl-4-(3-methoxy-4-amino-phenyl)-1H-imidazole | M.p. 167—168°C |
| 2-Phenyl-4-(4-amino-phenyl)-1H-imidazole | M.p. 198—199°C |

Example 3

2-Methylthio-4-(4-nitro-phenyl)-1H-imidazole

To a solution of sodium (2.3 g) in ethanol (190 ml) was added in portions 2-mercapto-4-(4-nitro-phenyl)-1H-imidazole (21.1 g). After 15 minutes stirring, a solution of iodomethane (14.2 g) in ethanol (50 ml) was dropped. After an additional 2 hours stirring at room temperature, the solution was evaporated to dryness. The residual solid was treated with water, filtered and dried to give 18.1 g of the title compound. M.p. 200—202°C.

2-Methoxy-4-(4-nitro-phenyl)-1H-imidazole (M.p. 295—300°C) was similarly prepared by this procedure starting from 2-hydroxy-4-(4-nitro-phenyl)-1H-imidazole.

Example 4

2-Methylthio-4-(4-amino-phenyl)-1H-imidazole

Concentrated hydrochloric acid (60 ml) was dropped into a stirred suspension of 2-methylthio-4-(4-nitro-phenyl)-1H-imidazole (5.88 g), granulated tin (8.9 g) in water (25 ml). After an additional 3 hours stirring at 100°C, the mixture was cooled to room temperature and filtered. The solid was dissolved in water, the solution was filtered and evaporated to dryness. The residual solid was washed with ethanol, filtered and treated with an ethanolic solution of sodium ethoxyde to give the free base (3 g). M.p. 85—86°C. Following the above procedure, other nitrophenyl derivatives were reduced to give the following amines

| | |
|---|---|
| 2-Chloro-4-(4-amino-phenyl)-1H-imidazole | M.p. 56—58°C |
| 2-Methylsulfinyl-4-(4-amino-phenyl)-1H-imidazole | M.p. 83—85°C |
| 2-Sulfamoyl-4-(4-amino-phenyl)-1H-imidazole | M.p. 111—113°C |

Example 5

2-Chloro-4-(4-nitro-phenyl)-1H-imidazole

A mixture of 2-hydroxy-4-(4-nitro-phenyl)-1H-imidazole (15 g) and phosphorus oxychloride (750 ml) was refluxed under stirring for 24 hours. The solution was evaporated to dryness, the residue was taken to pH 6.5 with a $H_2PO_4^-/HPO_4^{--}$ buffer, and extracted with methylene chloride. After drying, the organic solution was evaporated and the crude product was chromatographed on silica-gel to give 6.7 g of the title compound. M.p. 136—138°C.

Example 6

2-Methylsulfinyl-4-(4-nitro-phenyl)-1H-imidazole

Hydrogen peroxide (36%, 100 ml) was added in 2 hours under stirring to a hot (60°C) solution of 2-methylthio-4-(4-nitro-phenyl)-1H-imidazole (19.6 g) in methanol (500 ml). After an additional 24 hours heating at 60°C, the mixture was cooled to room temperature, filtered and the solid was dried to give 16.1 g of the title compound. M.p. 220—222°C.

Example 7

2-Sulfamoyl-4-(4-nitro-phenyl)-1H-imidazole

Chlorine was introduced through a capillary tube at a moderately rapid rate for 40 minutes into a stirred ice-cooled mixture of 2-mercapto-4-(4-nitro-phenyl)-1H-imidazole (10 g) in N hydrochloric acid (100 ml). The sulfonyl chloride which separated was filtered, washed with cold water and added to an excess of liquid ammonia. After evaporation of the excess ammonia, the residue was taken up in a little dilute ammonium hydroxide and filtered with charcoal. The solution was cooled and made slightly acidic, the solid which separated was filtered and recrystallized from water to give 3.4 g of the title compound. M.p. 227—229°C.

Example 8

2-Amino-4-(4-nitro-phenyl)-1H-imidazole

A mixture of α-amino-4-nitro-acetophenone hydrochloride (70 g), cyanamide (40.7 g), water (2100 ml), and conc. acetic acid (70 ml) was taken to pH 4.5 wih 30% sodium hydroxide and heated to 100°C for 45 minutes. The hot solution was filtered and cooled. The solid which separated was filtered, washed with water and dried to give 25 g of the title compound. M.p. 235—240°C (as hydrochloride).

Example 9

2-Acetamido-4-(4-nitro-phenyl)-1H-imidazole

A mixture of 2-Amino-4-(4-nitro-phenyl)-1H-imidazole (17.3 g) in acetic anhydride (100 ml) was heated at 100°C for 1 hour. The solution was evaporated to dryness, and the residue was treated with ether, filtered and dried to give 18.9 g of the title compound. M.p. 242—244°C.

Example 10

2-Ethylamino-4-(4-amino-phenyl)-1H-imidazole

A suspension of 2-acetamido-4-(4-amino-phenyl)-1H-imidazole (16 g) in anhydrous tetrahydrofuran (160 ml) was dropped into a stirred suspension of lithium aluminium hydride (12 g) in anhydrous tetrahydrofuran (240 ml). The mixture was heated at 60°C for 4 hours.

After cooling and treatment with water, the solvent was evaporated and the residue was dissolved in 10% hydrochloric acid. The solution was made alkaline with 30% sodium hydroxide and the base was extracted with ethyl acetate. The organic phase was evaporated to dryness to give 8.2 g of the title compound. M.p. 89—91°C.

Example 11

2-Methyl-4-(3-bromo-4-amino-phenyl)-1H-imidazole

A mixture of 2-methyl-4-(3-bromo-4-acetamido-phenyl)-1H-imidazole (15 g), concentrated hydrochloric acid (10 ml) and water (30 ml) was refluxed for 45 minutes. The solution was cooled to room temperature and made alkaline with 10% sodium hydroxide. The base was filtered, washed with water and dried to give 10 g of the title compound. M.p. 34—36°C.

Example 12

2-Methyl-4-(3-cyano-4-amino-phenyl)-1H-imidazole

A mixture of 2-methyl-4-(3-bromo-4-amino-phenyl)-1H-imidazole (5.12 g), copper (I) cyanide (2 g), and quinoline (15 ml) was refluxed for 4 hours. After cooling and decomposition with a acid solution of ferric chloride, the mixture was extracted with ethyl acetate. The organic solvent was dried and evaporated to dryness and the residue was treated with water, filtered and dried to give 2.1 g of the title compound. M.p. 89—91°C.

Example 13

2-Methyl-4-(3-carbamoyl-4-amino-phenyl)-1H-imidazole

A mixture of 2-methyl-4-(3-cyano-4-amino-phenyl)-1H-imidazole (6.2 g) and Amberlite IRA 400 (OH) (12 g) in water (150 ml) was refluxed for 5 hours. After cooling and filtration, the solution was evaporated to dryness to give 4.1 g of the title compound. M.p. 137—139°C.

Example 14

2-Methyl-4-(3-methyl-4-nitro-phenyl)-1H-imidazole

2-Methyl-4-(3-methyl-phenyl)-1H-imidazole as a nitrate salt (15 g) was added portionwise to 96% sulfuric acid (40 ml) at room temperature. The solution was heated at 90°C for 1 hour, cooled, diluted with water (200 ml) and neutralized with aqueous sodium carbonate. The solid which separated was filtered and the solution was made strongly alkaline with 10% sodium hydroxide. The base which precipitated was filtered and dried to give 5 g of the title compound sufficiently pure to be used in the next step. M.p. 158—160°C.

The following intermediate was prepared in a similar manner:

| | |
|---|---|
| *2-Methyl-4-(3-methoxy-4-nitro-phenyl)-1H-imidazole* | M.p. 191—192°C |

Example 15

N-Cyano-N′-[4-(2-methyl-imidazol-4-yl)phenyl]-formamidine

A solution of 2-methyl-4-(4-amino-phenyl)-1H-imidazole (15 g) and ethyl N-cyanoformimidate (9.35 g) in ethanol (150 ml) was stirred at room temperature overnight. The crystallized product was collected by filtration, washed with cold ethanol and dried to give 15.2 g of the title compound. M.p. 175—176°C (dec.).

Following the above procedure, using the appropriate starting materials, the following N-cyano-amidines were prepared:

| | |
|---|---|
| N-Cyano-N′-[3-(2-methyl-imidazol-4-yl)phenyl]-formamidine | M.p. 225—230°C (dec) |
| N-Cyano-N′-[4-(2-ethyl-imidazol-4-yl)phenyl]-formamidine | M.p. 227—229°C (dec) |
| N-Cyano-N′-[4-(2-isopropyl-imidazol-4-yl)phenyl]-formamidine | M.p. 221—223°C (dec) |
| N-Cyano-N′-[4-(2-methyl-imidazol-4-yl)phenyl]-acetamidine | M.p. 152—153°C (dec) |
| N-Cyano-N′-[4-(2,5-dimethyl-imidazol-4-yl)phenyl]-formamidine | M.p. 145—148°C (dec) |
| N-Cyano-N′-[4-(2-mercapto-imidazol-4-yl)phenyl]-formamidine M.p. | 270°C (dec) |
| N-Cyano-N′-[4-(2-methylmercapto-imidazol-4-yl)phenyl]-formamidine | M.p. 232—235°C (dec) |
| N-Cyano-N′-[4-(2-hydroxy-imidazol-4-yl)phenyl]-formamidine M.p. | 255—258°C (dec) |
| N-Cyano-N′-[4-(2-methoxy-imidazol-4-yl)phenyl]-formamidine | M.p. 270°C (dec) |
| N-Cyano-N′-[4-(2-sulfamoyl-imidazol-4-yl)phenyl]-formamidine | M.p. 241—242°C (dec) |
| N-Cyano-N′-[4-(2-acetamido-imidazol-4-yl)phenyl]-formamidine | M.p. 228—230°C (dec) |

| | |
|---|---|
| N-Cyano-N'-[4-(2-ethyl-amino-imidazol-4-yl)phenyl]-formamidine | M.p. 215—216°C (dec) |
| N-Cyano-N'-[2-bromo-4-(2-methyl-imidazol-4-yl)phenyl]-formamidine | M.p. 247—248°C (dec) |
| N-Cyano-N'-[2-cyano-4-(2-methyl-imidazol-4-yl)phenyl]-formamidine | M.p. 239—241°C (dec) |
| N-Cyano-N'-[2-carbamoyl-4-(2-methyl-imidazol-4-yl)phenyl]-formamidine | M.p. 250—252°C (dec) |
| N-Cyano-N'-[4-(2-amino-imidazol-4-yl)phenyl]-formamidine | M.p. 220—222°C (dec) |
| N-Cyano-N'-[2-methyl-4-(2-methyl-imidazol-4-yl)phenyl]-formamidine | M.p. 234—236°C (dec) |
| N-Cyano-N'-[2-methoxy-4-(2-methyl-imidazol-4-yl)phenyl]-formamidine | M.p. 263—266°C (dec) |
| N-Cyano-N'-[4-(2-phenyl-imidazol-4-yl)phenyl]-formamidine | M.p. >280°C |
| N-Cyano-N'-[4-(2-methyl-sulfinyl-imidazol-4-yl)phenyl]-formamidine | M.p. 222—224°C (dec) |

Example 16

N-Methyl-N'-[4-(2-methyl-imidazol-4-yl)phenyl]-formamidine (Compound 1)

N-Cyano-N'-[4-(2-methyl-imidazol-4-yl)phenyl]-formamidine (2.25 g) was added in one portion in a solution of methylamine 33% aqueous (22.5 ml). After few minutes the solid which separated was filtered, washed with water and dried to five 1.2 g of the title compound.

Maleate salt (ethanol) M.p. 173—174°C (dec)

Analysis

| | | | | |
|---|---|---|---|---|
| $C_{20}H_{22}N_4O_8$ | Found % | C 54.07 | H 4.79 | N 12.31 |
| | Calc. % | C 53.81 | H 4.97 | N 12.55 |

The following compounds were prepared in a similar manner starting from the N-cyanoamidine derivatives previously described:

N-Ethyl-N'-[4-(2-methyl-imidazol-4-yl)phenyl]-formamidine (Compound 2)

Maleate salt (ethanol) M.p. 166—168°C (dec)

Analysis

| | | | | |
|---|---|---|---|---|
| $C_{21}H_{24}N_4O_8$ | Found % | C 54.49 | H 5.21 | N 12.38 |
| | Calc. % | C 54.78 | H 5.25 | N 12.17 |

N-Isopropyl-N'-[4-(2-methyl-imidazol-4-yl)phenyl]-formamidine (Compound 3)

Maleate salt (ethanol) M.p. 192—193°C (dec)

Analysis

| | | | | |
|---|---|---|---|---|
| $C_{22}H_{26}N_4O_8$ | Found % | C 55.84 | H 5.59 | N 11.63 |
| | Calc. % | C 55.69 | H 5.52 | N 11.81 |

N-Allyl-N'-[4-(2-methyl-imidazol-4-yl)phenyl]-formamidine (Compound 4)

Maleate salt (ethanol) M.p. 170—171°C (dec)

Analysis

| | | | | |
|---|---|---|---|---|
| $C_{22}H_{24}N_4O_8$ | Found % | C 56.17 | H 5.00 | N 12.08 |
| | Calc. % | C 55.93 | H 5.12 | N 11.86 |

N-Propargyl-N'-[4-(2-methyl-imidazol-4-yl)phenyl]-formamidine (Compound 5)

Maleate salt (ethanol) M.p. 160—162°C (dec)

Analysis

| | | | | |
|---|---|---|---|---|
| $C_{22}H_{22}N_4O_8$ | Found % | C 56.23 | H 4.70 | N 12.05 |
| | Calc. % | C 56.17 | H 4.71 | N 11.91 |

N-n-Hexyl-N'-[4-(2-methyl-imidazol-4-yl)phenyl]-formamidine (Compound 6)

Maleate salt (acetone) M.p. 127—130°C (dec)

Analysis

| | | | | |
|---|---|---|---|---|
| $C_{25}H_{32}N_4O_8$ | Found % | C 57.87 | H 6.24 | N 10.62 |
| | Calc. % | C 58.13 | H 6.24 | N 10.85 |

N-(2-Methoxyethyl)-N'-[4-(2-methyl-imidazol-4-yl)phenyl]-formamidine (Compound 7)

Maleate salt (acetone) M.p. 161—163°C (dec)

Analysis

| | | | | |
|---|---|---|---|---|
| $C_{22}H_{26}N_4O_9$ | Found % | C 54.13 | H 5.29 | N 11.28 |
| | Calc. % | C 53.87 | H 5.34 | N 11.42 |

N-(3-Hydroxypropyl)-N'-[4-(2-methyl-imidazol-4-yl)phenyl]-formamidine (Compound 8)
Maleate salt (acetone) M.p. 166—169°C (dec)

| Analysis | | | | |
|---|---|---|---|---|
| $C_{22}H_{26}N_4O_9$ | Found % | C 53.57 | H 5.21 | N 11.59 |
| | Calc. % | C 53.87 | H 5.34 | N 11.42 |

N-(2-Methylthioethyl)-N'-[4-(2-methyl-imidazol-4-yl)phenyl]-formamidine (Compound 9)
Maleate salt (ethanol) M.p. 170—172°C (dec)

| Analysis | | | | |
|---|---|---|---|---|
| $C_{22}H_{26}N_4O_8S$ | Found % | C 51.99 | H 5.27 | N 11.30 |
| | Calc. % | C 52.17 | H 5.18 | N 11.06 |

N-(2-Cyanoethyl)-N'-[4-(2-methyl-imidazol-4-yl)phenyl]-formamidine (Compound 10)
Maleate salt (ethanol) M.p. 162—164°C (dec)

| Analysis | | | | |
|---|---|---|---|---|
| $C_{22}H_{23}N_5O_8$ | Found % | C 54.29 | H 4.71 | N 14.35 |
| | Calc. % | C 54.43 | H 4.78 | N 14.43 |

N-Cyclopropylmethyl-N'-[4-(2-methyl-imidazol-4-yl)phenyl]-formamidine (Compound 11)
Fumarate salt (ethanol) M.p. 154—157°C (dec)

| Analysis | | | | |
|---|---|---|---|---|
| $C_{23}H_{26}N_4O_8$ | Found % | C 56.60 | H 5.30 | N 11.53 |
| | Calc. % | C 56.78 | H 5.39 | N 11.52 |

N-Cyclohexyl-N'-[4-(2-methyl-imidazol-4-yl)phenyl]-formamidine (Compound 12)
Fumarate salt (ethanol) M.p. 164—166°C (dec)

| Analysis | | | | |
|---|---|---|---|---|
| $C_{25}H_{30}N_4O_8$ | Found % | C 58.07 | H 5.95 | N 11.00 |
| | Calc. % | C 58.36 | H 5.88 | N 10.89 |

N-Benzyl-N'-[4-(2-methyl-imidazol-4-yl)phenyl]-formamidine (Compound 13)
Hydrochloride salt (ethanol) M.p. 225—227°C (dec)

| Analysis | | | | |
|---|---|---|---|---|
| $C_{18}H_{20}Cl_2N_4$ | Found % | C 59.39 | H 5.65 | N 15.63 |
| | Calc. % | C 59.51 | H 5.55 | N 15.42 |

N-Furfuryl-N'-[4-(2-methyl-imidazol-4-yl)phenyl]-formamidine (Compound 14)
Hydrochloride salt (ethanol) M.p. 235—237°C (dec)

| Analysis | | | | |
|---|---|---|---|---|
| $C_{16}H_{18}Cl_2N_4O$ | Found % | C 54.67 | H 5.27 | N 15.68 |
| | Calc. % | C 54.40 | H 5.14 | N 15.86 |

N-Ethyl-N'-[4-(2-ethyl-imidazol-4-yl)phenyl]-formamidine (Compound (15)
Maleate salt (ethanol) M.p. 165—167°C (dec)

| Analysis | | | | |
|---|---|---|---|---|
| $C_{22}H_{26}N_4O_8$ | Found % | C 55.78 | H 5.51 | N 11.59 |
| | Calc. % | C 55.69 | H 5.52 | N 11.81 |

N-Isopropyl-N'-[4-(2-ethyl-imidazol-4-yl)phenyl]-formamidine (Compound 16)
Maleate salt (ethanol) M.p. 180—181°C (dec)

| Analysis | | | | |
|---|---|---|---|---|
| $C_{23}H_{28}N_4O_8$ | Found % | C 56.95 | H 5.79 | N 11.30 |
| | Calc. % | C 56.55 | H 5.78 | N 11.47 |

N-Ethyl-N'-[4-(2-isopropyl-imidazol-4-yl)phenyl]-formamidine (Compound 17)
Maleate salt (ethanol) M.p. 174—175°C (dec)

| Analysis | | | | |
|---|---|---|---|---|
| $C_{23}H_{28}N_4O_8$ | Found % | C 56.73 | H 5.87 | N 11.60 |
| | Calc. % | C 56.55 | H 5.78 | N 11.47 |

N-Isopropyl-N'-[4-(2-isopropyl-imidazol-4-yl)phenyl]-formamidine (Compound 18)
Maleate salt (ethanol) M.p. 168—170°C (dec)

| Analysis | | | | |
|---|---|---|---|---|
| $C_{24}H_{30}N_4O_8$ | Found % | C 57.02 | H 6.10 | N 11.28 |
| | Calc. % | C 57.36 | H 6.02 | N 11.15 |

N-Isopropyl-N'-[4-(2-phenyl-imidazol-4-yl)phenyl]-formamidine (Compound 19)
Maleate salt (ethanol) M.p. 208—210°C (dec)

| Analysis | | | | |
|---|---|---|---|---|
| $C_{23}H_{24}N_4O_4$ | Found % | C 66.01 | H 5.95 | N 13.20 |
| | Calc. % | C 65.70 | H 5.75 | N 13.33 |

N-Isopropyl-N'-[4-(2-methyl-imidazol-4-yl)phenyl]-acetamidine (Compound 20)
Maleate salt (ethanol) M.p. 106—108°C (dec)

| Analysis | | | | |
|---|---|---|---|---|
| $C_{23}H_{28}N_4O_8$ | Found % | C 56.67 | H 5.76 | N 11.55 |
| | Calc. % | C 56.55 | H 5.78 | N 11.47 |

N-Ethyl-N'-[4-(2,5-dimethyl-imidazol-4-yl)phenyl]-formamidine (Compound 21)
Maleate salt (acetone) M.p. 165—167°C (dec)

| Analysis | | | | |
|---|---|---|---|---|
| $C_{22}H_{26}N_4O_8$ | Found % | C 55.41 | H 5.70 | N 12.01 |
| | Calc. % | C 55.69 | H 5.52 | N 11.81 |

N-Isopropyl-N'-[4-(2,5-dimethyl-imidazol-4-yl)phenyl]-formamidine (Compound 22)
Maleate salt (acetone) M.p. 157—159°C (dec)

| Analysis | | | | |
|---|---|---|---|---|
| $C_{23}H_{28}N_4O_8$ | Found % | C 56.38 | H 5.82 | N 11.59 |
| | Calc. % | C 56.55 | H 5.78 | N 11.47 |

N-Ethyl-N'-[3-(2-methyl-imidazol-4-yl)phenyl]-formamidine (Compound 23)
Maleate salt (acetone) M.p. 171—173°C (dec)

| Analysis | | | | |
|---|---|---|---|---|
| $C_{21}H_{24}N_4O_8$ | Found % | C 54.61 | H 5.19 | N 11.98 |
| | Calc. % | C 54.78 | H 5.25 | N 12.17 |

N-Isopropyl-N'-[3-(2-methyl-imidazol-4-yl)phenyl]-formamidine (Compound 24)
Maleate salt (acetone) M.p. 168—171°C (dec)

| Analysis | | | | |
|---|---|---|---|---|
| $C_{22}H_{26}N_4O_8$ | Found % | C 55.44 | H 5.48 | N 11.69 |
| | Calc. % | C 55.69 | H 5.52 | N 11.81 |

N-Isopropyl-N'-[4-(2-mercapto-imidazol-4-yl)phenyl]-formamidine (Compound 25)
Maleate salt (acetone) M.p. 202°C (dec)

| Analysis | | | | |
|---|---|---|---|---|
| $C_{17}H_{20}N_4O_4S$ | Found % | C 54.38 | H 5.33 | N 14.70 |
| | Calc. % | C 54.24 | H 5.35 | N 14.88 |

N-Ethyl-N'-[4-(2-methylmercapto-imidazol-4-yl)phenyl]-formamidine (Compound 26)
Maleate salt (ethanol) M.p. 158—160°C (dec)

| Analysis | | | | |
|---|---|---|---|---|
| $C_{21}H_{24}N_4O_8S$ | Found % | C 51.31 | H 5.00 | N 11.28 |
| | Calc. % | C 51.22 | H 4.91 | N 11.38 |

N-Isopropyl-N'-[4-(2-methylmercapto-imidazol-4-yl)phenyl]-formamidine (Compound 27)
Maleate salt (acetone) M.p. 150—152°C (dec)

| Analysis | | | | |
|---|---|---|---|---|
| $C_{22}H_{26}N_4O_8S$ | Found % | C 51.93 | H 5.20 | N 11.25 |
| | Calc. % | C 52.17 | H 5.18 | N 11.06 |

N-Isopropyl-N'-[4-(2-hydroxy-imidazol-4-yl)phenyl]-formamidine (Compound 28)
Maleate salt (acetone) M.p. 211—213°C (dec)

| Analysis | | | | |
|---|---|---|---|---|
| $C_{17}H_{20}N_4O_5$ | Found % | C 56.47 | H 5.69 | N 15.58 |
| | Calc. % | C 56.66 | H 5.59 | N 15.55 |

N-Isopropyl-N'-[4-(2-methylsulfinyl-imidazol-4-yl)phenyl]-formamidine (Compound 29)
Base (water) M.p. 200—201°C (dec)

| Analysis | | | | |
|---|---|---|---|---|
| $C_{14}H_{18}N_3O\ S$ | Found % | C 57.82 | H 6.23 | N 19.15 |
| | Calc. % | C 57.92 | H 6.25 | N 19.30 |

N-Isopropyl-N'-[4-(2-sulfamoyl-imidazol-4-yl)phenyl]-formamidine (Compound 30)
Maleate salt (acetone) M.p. 243—246°C (dec)

| Analysis | | | | |
|---|---|---|---|---|
| $C_{17}H_{21}N_5O_6S$ | Found % | C 47.97 | H 5.07 | N 16.63 |
| | Calc. % | C 48.22 | H 5.00 | N 16.54 |

N-Isopropyl-N'-[4-(2-amino-imidazol-4-yl)phenyl]-formamidine (Compound 31)
Maleate salt (ethanol) M.p. 212—215°C (dec)

| Analysis | | | | |
|---|---|---|---|---|
| $C_{21}H_{25}N_5O_8$ | Found % | C 52.80 | H 5.23 | N 14.81 |
| | Calc. % | C 53.05 | H 5.30 | N 14.73 |

N-Isopropyl-N'-[4-(2-acetamido-imidazol-4-yl)phenyl]-formamidine (Compound 32)
Maleate salt (ethanol) M.p. 196—198°C (dec)

| Analysis | | | | |
|---|---|---|---|---|
| $C_{23}H_{27}N_5O_9$ | Found % | C 53.40 | H 5.22 | N 13.44 |
| | Calc. % | C 53.38 | H 5.26 | N 13.53 |

N-Isopropyl-N'-[4-(2-ethylamino-imidazol-4-yl)phenyl]-formamidine (Compound 33)
Maleate salt (ethanol) M.p. 225—227°C (dec)

| Analysis | | | | |
|---|---|---|---|---|
| $C_{23}H_{29}N_5O_8$ | Found % | C 54.50 | H 5.73 | N 14.09 |
| | Calc. % | C 54.86 | H 5.81 | N 13.91 |

N-n-Propyl-N'-[2-bromo-4-(2-methyl-imidazol-4-yl)phenyl]-formamidine (Compound 34)
Maleate salt (acetone) M.p. 186—188°C (dec)

| Analysis | | | | |
|---|---|---|---|---|
| $C_{22}H_{25}BrN_4O_8$ | Found % | C 47.66 | H 4.60 | N 10.01 |
| | Calc. % | C 47.75 | H 4.55 | N 10.12 |

N-Isopropyl-N'-[2-cyano-4-(2-methyl-imidazol-4-yl)phenyl]-formamidine (Compound 35)
Maleate salt (ethanol) M.p. 178—180°C (dec)

| Analysis | | | | |
|---|---|---|---|---|
| $C_{23}H_{25}N_5O_8$ | Found % | C 55.12 | H 4.99 | N 13.88 |
| | Calc. % | C 55.30 | H 5.05 | N 14.02 |

N-Ethyl-N'-[2-carbamoyl-4-(2-methyl-imidazol-4-yl)phenyl]-formamidine (Compound 36)
Maleate salt (acetone) M.p. 233—235°C (dec)

| Analysis | | | | |
|---|---|---|---|---|
| $C_{22}H_{25}N_5O_9$ | Found % | C 52.63 | H 5.09 | N 14.10 |
| | Calc. % | C 52.48 | H 5.01 | N 13.91 |

N-Allyl-N'-[2-methyl-4-(2-methyl-imidazol-4-yl)phenyl]-formamidine (Compound 37)
Maleate salt (acetone) M.p. 170—172°C (dec)

| Analysis | | | | |
|---|---|---|---|---|
| $C_{23}H_{26}N_4O_8$ | Found % | C 57.00 | H 5.45 | N 11.38 |
| | Calc. % | C 56.78 | H 5.39 | N 11.52 |

N-n-Propyl-N'-[2-methoxy-4-(2-methyl-imidazol-4-yl)phenyl]-formamidine (Compound 38)
Maleate salt (acetone) M.p. 201—203°C (dec)

| Analysis | | | | |
|---|---|---|---|---|
| $C_{22}H_{28}N_4O_9$ | Found % | C 54.44 | H 5.59 | N 10.97 |
| | Calc. % | C 54.75 | H 5.59 | N 11.11 |

Example 17

N-tert-Butyl-N'-[4-(2-methyl-imidazol-4-yl)phenyl]-formamidine (Compound 39)

To a solution N-tert-butyl formamide (5.06 g) in dichloromethane (30 ml) was slowly added at 0°C a solution of triethyloxonium fluoroborate (9.56 g) in dichloromethane (50 ml). After 6 hours stirring at room temperature, 2-methyl-4-(4-amino-phenyl)-1H-imidazole (4 g) in ethanol (20 ml) was added dropwise.

The mixture was stirred overnight, and then evaporated to dryness. The fluoborate salt of the desired compound was dissolved in water and the solution was made alkaline with 10% sodium hydroxide. The product which separated was filtered, washed with water and dried to five 2.1 g of the title formamidine.

Sulphate salt (methanol) M.p. 276—278°C (dec)

| Analysis | | | | |
|---|---|---|---|---|
| $C_{15}H_{22}N_4O_4S$ | Found % | C 50.79 | H 6.31 | N 15.77 |
| | Calc. % | C 50.84 | H 6.26 | N 15.81 |

Example 18

N-Phenyl-N'-[4-(2-methyl-imidazol-4-yl)phenyl]-formamidine (Compound 40)

2-Methyl-4-(4-amino-phenyl)-1H-imidazole (5 g) was added to a solution of ethyl N-phenyl formimidate (5 g) in acetone (25 ml). After 3 hours stirring at room temperature, the solid which separated was filtered and dried. The compound was treated with glac. acetic acid in acetone: the acetate salt which precipitated was filtered and dried to five 4.6 g of the title compound.

Acetate salt (acetone) M.p. 147—150°C (dec)

| Analysis | | | | |
|---|---|---|---|---|
| $C_{21}H_{24}N_4O_4$ | Found % | C 63.77 | H 6.13 | N 14.27 |
| | Calc. % | C 63.62 | H 6.10 | N 14.13 |

The following compounds were prepared in a similar manner starting from the appropriate ethyl formimidates:

N-(4-Chlorophenyl)-N'-[4-(2-methyl-imidazol-4-yl)phenyl]-formamidine (Compound 41)
Acetate salt (acetone) M.p. 112—115°C (dec)

| Analysis | | | | |
|---|---|---|---|---|
| $C_{21}H_{23}ClN_4O_4$ | Found % | C 58.29 | H 5.60 | N 13.12 |
| | Calc. % | C 58.54 | H 5.38 | N 13.00 |

N-(2-Pyridyl)-N'-[4-(2-methyl-imidazol-4-yl)phenyl]-formamidine (Compound 42)
Base (acetone) M.p. 59—61°C (dec)

| Analysis | | | | |
|---|---|---|---|---|
| $C_{16}H_{15}N_5$ | Found % | C 68.99 | H 5.30 | N 25.36 |
| | Calc. % | C 69.29 | H 5.45 | N 25.26 |

N-Isopropyl-N'-[4-(2-chloro-imidazol-4-yl)phenyl]-formamidine (Compound 43)
Base (acetone) M.p. 85—87°C (dec)

| Analysis | | | | |
|---|---|---|---|---|
| $C_{12}H_{13}ClN_4$ | Found % | C 57.89 | H 5.33 | N 22.41 |
| | Calc. % | C 57.95 | H 5.27 | N 22.53 |

N-Isopropyl-N'-[4-(2-methoxy-imidazol-4-yl)phenyl]-formamidine (Compound 44)
Maleate salt (methanol) M.p. 236°C (dec)

| Analysis | | | | |
|---|---|---|---|---|
| $C_{22}H_{26}N_4O_9$ | Found % | C 54.02 | H 5.46 | N 11.53 |
| | Calc. % | C 53.87 | H 5.34 | N 11.42 |

The following not limitative examples of pharmaceutical compositions according to the invention are reported:

Example 19

Tablets

| | |
|---|---|
| — active ingredient | 50 mg |
| — lactose | 217 mg |
| — corn starch | 30 mg |
| — magnesium stearate | 3 mg |

Method of preparation: the active ingredient, lactose, and corn starch were mixed and homogeneously moistened with water. After screening of the moist mass and drying in a tray driver, the mixture was again passed through a screen and magnesium stearate was added. Then the mixture was pressed into tablets weighing 300 mg each. Each tablet contains 50 mg of active ingredient.

Example 20

Capsules

| | |
|---|---|
| — active ingredient | 50 mg |
| — corn starch | 170 mg |
| — magnesium stearate | 2 mg |

Method of preparation: the active ingredient was mixed with the auxiliary products, and the mixture was passed through a screen and mixed homogeneously in a suitable device. The resulting mixture was filled into hard gelatine capsule (222 mg per capsule); each capsule contains 50 mg of active ingredient.

Example 21

Vials

| | |
|---|---|
| — active ingredient | 50 mg |
| — water for injection q.s. | 5 ml |

Method of preparation: the active ingredient was dissolved in the water in appropriate amounts, and then the resulting solution was introduced into 5 ml vials under sterile conditions. Each vial contains 50 mg of active ingredient.

**Claims for the Contracting States: BE CH DE FR IT LI LU NL SE**

1. Compounds of general formula I

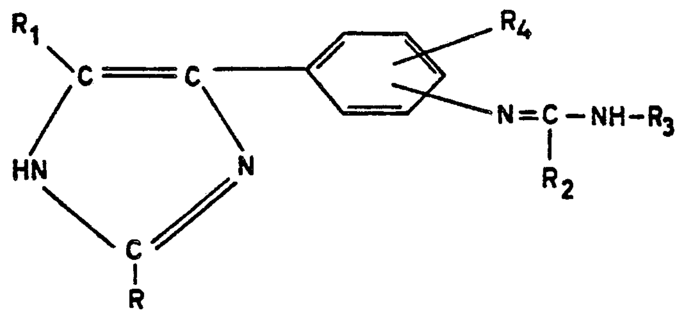

(I)

in which R represents a linear or branched $C_{1-4}$ alkyl group, a hydroxy group, a $C_{1-3}$ alkoxy group, a mercapto, a $C_{1-3}$ alkylthio group, a halogen atom, a $C_{1-3}$ alkylsulfinyl or $C_{1-3}$ alkylsulphonyl group, a sulfamoyl group, an amino group which may be substituted by one or two alkyl groups having 1 to 3 carbon atoms, a $C_{1-4}$alkanoylamino or phenyl group; $R_1$ and $R_2$ which may be the same or different, represent a hydrogen atom or a $C_{1-4}$alkyl group; $R_3$ represents a linear or branched $C_{1-8}$alkyl, hydroxy-($C_{1-6}$)alkyl, ($C_{1-6}$)alkoxy-($C_{1-6}$)alkyl, ($C_{1-6}$)alkylthio-($C_{1-6}$)alkyl, cyano-($C_{1-6}$)alkyl, a $C_{3-5}$alkenyl group, an alkynyl group having 3 or 4 carbon atoms, a cyano group, an aryl group optionally substituted by halogen, a benzyl group, a ($C_{3-6}$)cycloalkyl group, a ($C_{3-6}$)cycloalkyl+($C_{1-2}$)alkyl group, an aromatic 5- to 6-membered heterocyclic ring containing one hetero atom; $R_4$ represents a hydrogen atom, a $C_{1-3}$alkyl or $C_{1-3}$alkoxy group, a halogen atom, a cyano or carbamoyl group, tautomeric forms thereof and acid addition salts thereof.

2. Physiologically compatible acid addition salts of compounds of formula (I) as claimed in claim 1.

3. Salts as claimed in claim 2 formed with hydrochloric, sulphuric, maleic or fumaric acid.

4. N-Ethyl-N'-[4-(2-methyl-imidazol-4-yl)phenyl]formamidine.

5. N-Isopropyl-N'-[4-(2-methyl-imidazol-4-yl)phenyl]formamidine.

6. N-Allyl-N'-[4-(2-methyl-imidazol-4-yl)phenyl]formamidine.

7. N-t.Butyl-N'-[4-(2-methyl-imidazol-4-yl)phenyl]formamidine.

8. N-Isopropyl-N'-[4-(2-hydroxy-imidazol-4-yl)phenyl]formamidine.

9. Physiologically compatible acid addition salts of compounds as claimed in claims 4 to 8.

10. Maleic, hydrochloric, fumaric or sulphuric acid addition salts of compounds as claimed in claims 4 to 8.

11. A process for the preparation of compounds of general formula (I) as claimed in claim 1 which comprises reacting a compound of formula II

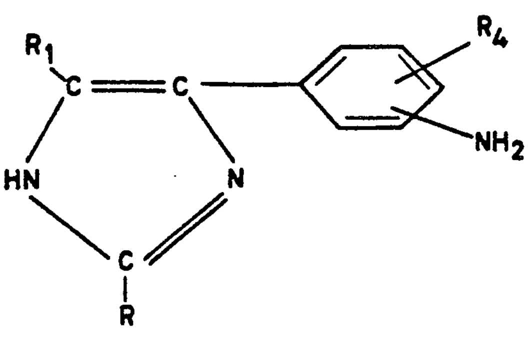

(II)

wherein R, $R_1$ and $R_4$ are as defined in claim 1, with a compound of formula III

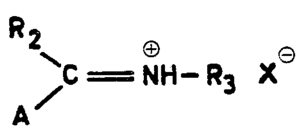

(III)

in which $R_2$ and $R_3$ are as defined in claim 1, $X^{\ominus}$ represents the anion of an inorganic acid and A represents a benzoyloxy group, chlorine or ethoxy group.

12. A process as claimed in claim 11 in which the anion of an inorganic acid comprises hydrochloride or fluoroborate.

13. A process as claimed in claim 11 in which a compound of formula (III) is made to react as a base.

14. A process as claimed in claims 11 to 13 in which the reaction is carried out in the presence of an inert organic solvent.

15. A process as claimed in claim 14 in which the inert organic solvent comprises ethanol, dioxane or acetone.

16. A process as claimed in claims 11 to 15 in which the reaction is carried out at a temperature of from 20 to 60°C.

17. A process for the preparation of compounds of general formula (I) as claimed in claim 1 which comprises reacting a compound of formula V

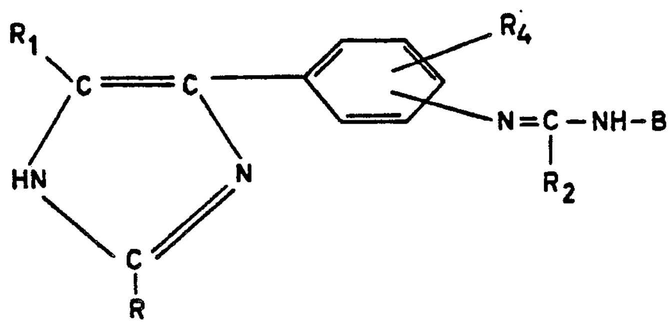

(V)

in which R, $R_1$, $R_2$ and $R_4$ are as defined in claim 1, B represents a suitable removable group, with an amine of formula VI

$$H_2N—R_3 \quad (VI)$$

in which $R_3$ is as defined in claim 1.

18. A process as claimed in claim 17 in which the suitable leaving group comprises a cyano, acetyl, ethoxycarbonyl or carbamyl group.

19. A process as claimed in claims 17 and 18 in which the reaction is carried out in the presence of water.

20. A process as claimed in claims 17 to 19 in which the reaction is carried out at room temperature.

21. Pharmaceutical compositions comprising as active ingredient at least one compound of general formula (I) as defined in claim 1 or a tautomer or physiologically compatible acid addition salt thereof, in association with a pharmaceutical carrier or excipient.

22. Pharmaceutical compositions as claimed in claim 21 for use as anti-ulcers.

23. Pharmaceutical compositions as claimed in claim 21 for the treatment of patients suffering from disorders of gastrointestinal tract.

24. Intermediate compound of formula V

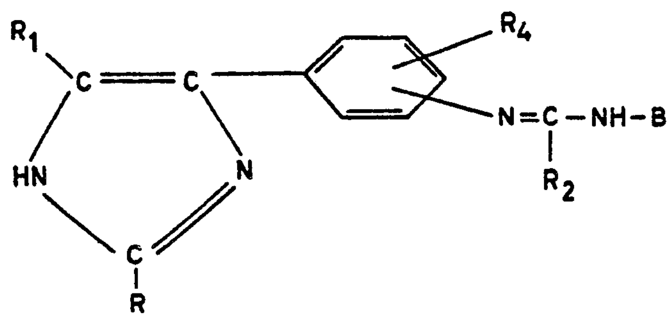

(V)

wherein R, $R_1$, $R_2$ and $R_4$ are as defined in claim 1 and B is as defined in claim 17 for the performance of the process as claimed in claim 17.

**Claims for the Contracting State: AT**

1. Process for the preparation of

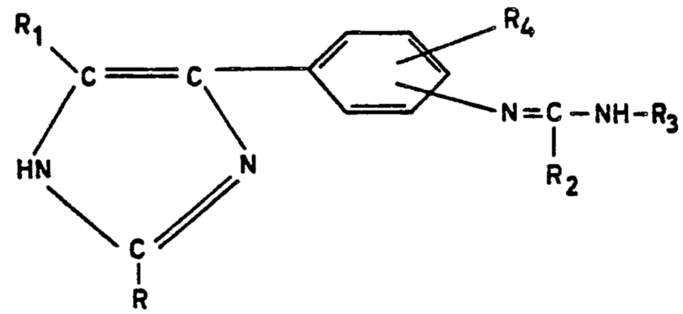

(I)

in which R represents a linear or branched $C_{1-4}$ alkyl group, a hydroxy group, a $C_{1-3}$ alkoxy group, a mercapto, a $C_{1-3}$ alkylthio group, a halogen atom, a $C_{1-3}$ alkylsulfinyl or $C_{1-3}$ alkylsulphonyl group, a sulfamoyl group, an amino group which may be substituted by one or two alkyl groups having 1 to 3 carbon atoms, a $C_{1-4}$alkanoylamino or phenyl group; $R_1$ and $R_2$ which may be the same or different, represent a hydrogen atom or a $C_{1-4}$alkyl group; $R_3$ represents a linear or branched $C_{1-8}$alkyl, hydroxy-$(C_{1-6})$alkyl, $(C_{1-6})$alkoxy-$(C_{1-6})$alkyl, $(C_{1-6})$alkylthio-$(C_{1-6})$alkyl, cyano-$(C_{1-6})$alkyl, a $C_{3-5}$alkenyl group, an alkynyl group having 3 or 4 carbon atoms, a cyano group, an aryl group optionally substituted by halogen, a benzyl group, a $(C_{3-6})$cycloalkyl group, a $(C_{3-6})$cycloalkyl+$(C_{1-2})$alkyl group, an aromatic 5- to 6-membered heterocyclic ring containing one hetero atom; $R_4$ represents a hydrogen atom, a $C_{1-3}$alkyl or $C_{1-3}$alkoxy group, a halogen atom, a cyano or carbamoyl group, tautomeric forms thereof and acid addition salts thereof, and physiologically compatible acid addition salts thereof which comprises reacting a compound of formula II

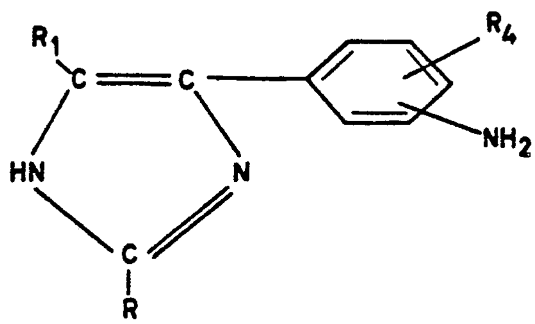

(II)

wherein R, $R_1$ and $R_4$ are as defined in claim 1, with a compound of formula III

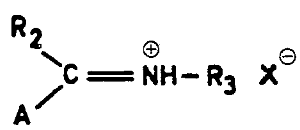

(III)

in which $R_2$ and $R_3$ are as defined in claim 1, $X^{\ominus}$ represents the anion of an inorganic acid and A represents a benzoyloxy group, chlorine or ethoxy group.

2. A process as claimed in claim 1, in which the anion of an inorganic acid comprises hydrochloride or fluoroborate.

3. A process as claimed in claim 1, in which a compound of formula (III) is made to react as a base.

4. A process as claimed in claims 1 to 3, in which the reaction is carried out in the presence of an inert organic solvent.

5. A process as claimed in claim 4, in which the inert organic solvent comprises ethanol, dioxane or acetone.

6. A process as claimed in claims 1 to 5, in which the reaction is carried out at a temperature of from 20 to 60°C.

7. A process for the preparation of compounds of general formula (I) as specified in claim 1 which comprises reacting a compound of formula V

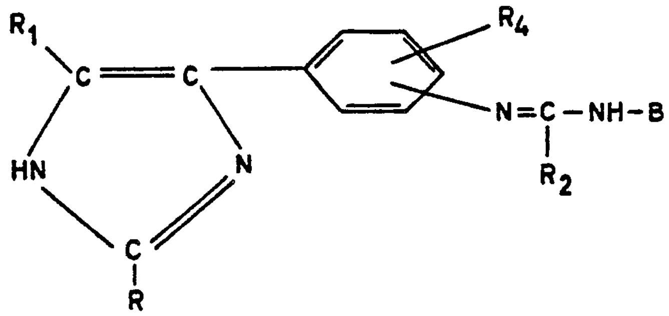

(V)

in which R, $R_1$, $R_2$ and $R_4$ are as defined in claim 1, B represents a suitable removable group, with an amine of formula VI

$$H_2N—R_3 \quad (VI)$$

in which $R_3$ is as defined in claim 1.

8. A process as claimed in claim 7, in which the suitable leaving group comprises a cyano, acetyl, ethoxycarbonyl or carbamyl group.

9. A process as claimed in claims 7 to 8, in which the reaction is carried out in the presence of water.

10. A process as claimed in claims 7 to 9, in which the reaction is carried out at room temperature.

11. A process as claimed in claim 7, wherein said salts are formed with hydrochloric, sulphuric, maleic or fumaric acid.

12. A process as claimed in claim 1, wherein said compound of formula (I) is: N-Ethyl-N'-[4-(2-methyl-imidazol-4-yl)phenyl]formamidine.

13. A process as claimed in claim 1, wherein said compound of formula (I) is: N-Isopropyl-N'-[4-(2-methyl-imidazol-4-yl)phenyl]formamidine.

14. A process as claimed in claim 1, wherein said compound of formula (I) is: N-Allyl-N'-[4-(2-methyl-imidazol-4-yl)phenyl]formamidine.

15. A process as claimed in claim 1, wherein said compound of formula (I) is: N-t.Butyl-N'-[4-(2-methyl-imidazol-4-yl)phenyl]formamidine.

16. A process as claimed in claim 1, wherein said compound of formula (I) is: N-Isopropyl-N'-[4-(2-hydroxy-imidazol-4-yl)phenyl]formamidine.

17. A process as claimed in claim 1, wherein said acid addition salts are: maleic, hydrochloric, fumaric or sulphuric acid addition salts.

18. A process for the preparation of a new intermediate compound of formula V

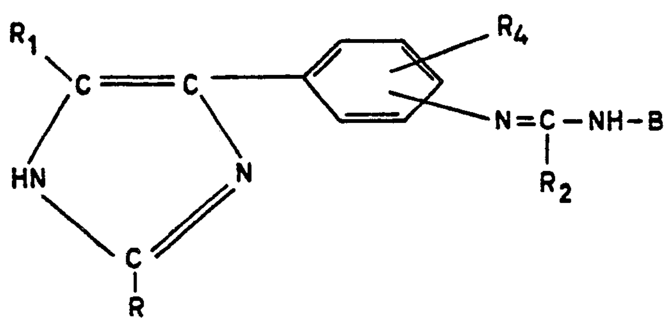

(V)

wherein R, $R_1$, $R_2$ and $R_4$ are as defined in claim 1 and B is as defined in claim 7 for the performance of the process as claimed in claim 7.

**Patentansprüche für die Vertragsstaaten: BE CH DE FR IT LI LU NL SE**

1. Verbindungen der allgemeinen Formel I

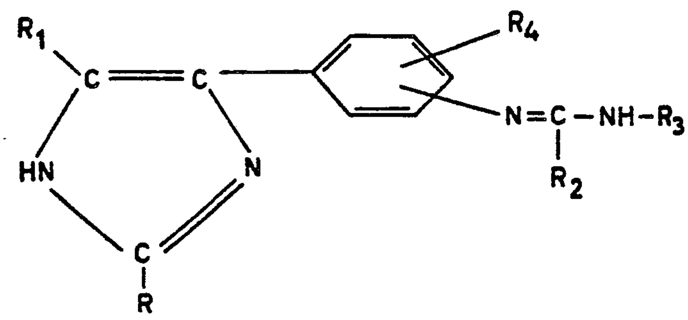

(I)

in welchen R eine geradkettige oder verzweigte $C_{1-4}$ Alkylgruppe, eine Hydroxygruppe, eine $C_{1-3}$ Alkoxygruppe, eine Mercapto-, eine $C_{1-3}$ Alkylthiogruppe, eine Halogenatom, eine $C_{1-3}$ Alkylsulfinyl- oder $C_{1-3}$ Alkylsulphonylgruppe, eine Sulfamoylgruppe, eine Aminogruppe, welche durch eine oder zwei Alkylgruppen mit ein bis drei Kohlenstoffatomen substituiert sein kann, eine $C_{1-4}$ Alkanoylaminogruppe oder Phenylgruppe bedeutet; $R_1$ und $R_2$, welche gleich oder voneinander verschieden sein können, jeweils ein Wasserstoffatom oder eine $C_{1-4}$ Alkylgruppe bedeuten; $R_3$ eine geradkettige oder verzweigte $C_{1-8}$ Alkyl-, Hydroxy-$(C_{1-6})$alkyl-, $(C_{1-6})$ Alkoxy-$(C_{1-6})$alkyl-, $(C_{1-6})$ Alkylthio-$(C_{1-6})$alkyl-, Cyano $(C_{1-6})$alkyl-, eine $C_{3-5}$ Alkenylgruppe, eine Alkynylgruppe mit 3 oder 4 Kohlenstoffatomen, eine Cyanogruppe, eine Arylgruppe, welche gegebenenfalls durch Halogen substituiert sein kann, eine Benzylgruppe, eine $(C_{3-6})$ Cycloalkylgruppe, eine $(C_{3-6})$ Cycloalkyl-$(C_{1-2})$alkylgruppe, eine aromatischer 5- bis 6-gliedriger heterocyclischer Ring, welcher ein Heteroatom enthält, bedeutet; $R_4$ ein Wasserstoffatom, eine $C_{1-3}$ Alkyl- oder $C_{1-3}$ Alkoxygruppe, ein Halogenatom, eine Cyano- oder Carbamoylgruppe bedeutet, tautomere Formen davon und Säureadditionssalze davon.

2. Physiologisch verträgliche Säureadditionssalze der Verbindungen der Formel (I) nach Anspruch 1.

3. Salze nach Anspruch 2, welche mit Chlorwasserstoff-, Schwefel-, Malein- und Fumarsäure gebildet werden.

4. N-Ethyl-N'-[4-(2-methyl-imidazol-4-yl)phenyl]formamidin.

5. N-Isopropyl-N'-[4-(2-methyl-imidazol-4-yl)phenyl]formamidin.

6. N-Allyl-N'-[4-(2-methyl-imidazol-4-yl)phenyl]formamidin.

7. N-tert.-Butyl-N'-[4-(2-methyl-imidazol-4-yl)phenyl]formamidin.

8. N-Isopropyl-N'-[4-(2-hydroxy-imidazol-4-yl)phenyl]formamidin.

9. Physiologisch verträgliche Säureadditionssalze der Verbindungen nach den Ansprüchen 4 bis 8.

10. Malein-, Chlorwasserstoff-, Fumar- oder Schwefelsäureadditionssalze der Verbindungen nach den Ansprüchen 4 bis 8.

11. Verfahren zur Herstellung von Verbindungen der allgemeinen Formel (I) nach Anspruch 1, welches die Umsetzung einer Verbindung der Formel II

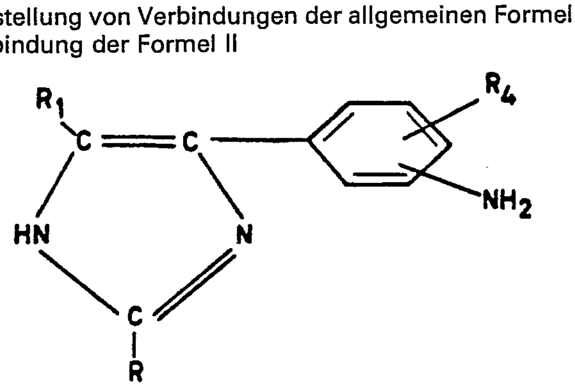

(II)

worin R, $R_1$ und $R_4$ wie in Anspruch 1 definiert sind, mit einer Verbindung der Formel III

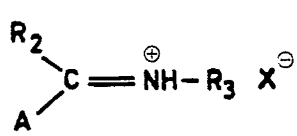

(III)

in welcher $R_2$ und $R_3$ wie in Anspruch 1 definiert sind, $X^{\ominus}$ ein Anion einer anorganischen Säure bedeutet und A eine Benzyloxygruppe, Chlor oder Ethoxygruppe bedeutet, umfaßt.

12. Verfahren nach Anspruch 11, in welchem das Anion einer anorganischen Säure Hydrochlorid oder Fluorborat bedeutet.

13. Verfahren nach Anspruch 11, bei welchem die Verbindung der Formel (III) als Base reagiert.

14. Verfahren nach einem der Ansprüche 11 bis 13, bei welchem die Reaktion in Gegenwart eines inerten organischen Lösungsmittels durchgeführt wird.

15. Verfahren nach Anspruch 14, bei welchem das inerte organische Lösungsmittel Ethanol, Dioxan oder Aceton umfaßt.

16. Verfahren nach einem der Ansprüche 11 bis 15, bei welchem die Reaktion bei einer Temperatur von 20 bis 60°C durchgeführt wird.

17. Verfahren zur Herstellung von Verbindungen der allgemeinen Formel (I) nach Anspruch 1, welches die Umsetzung einer Verbindung der Formel V

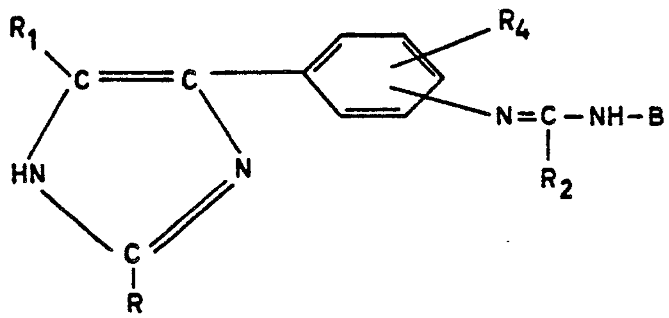

(V)

in welcher R, $R_1$, $R_2$ und $R_4$ wie in Anspruch 1 definiert sind, B eine geeignete Abgangsgruppe bedeutet, mit einem Amin der Formel VI

$$H_2N—R_3 \qquad (VI)$$

in welcher $R_3$ wie in Anspruch 1 definiert ist, umfaßt.

18. Verfahren nach Anspruch 17, bei welchem die geeignete Abgangsgruppe eine Cyano-, Acetyl-, Ethoxycarbonyl- oder Carbamylgruppe umfaßt.

19. Verfahren nach Anspruch 17 und 18, bei welchem die Reaktion in Gegenwart von Wasser durchgeführt wird.

20. Verfahren nach einem der Ansprüche 17 bis 19, bei welchem die Reaktion bei Raumtemperatur durchgefühurt wird.

21. Pharmazeutische Zubereitungen, welche als Wirkstoff zumindest eine Verbindung der allgemeinen Formel (I) nach Anspruch 1 oder ein Tautomer oder physiologisch verträgliches Säureadditionssalz davon, gemeinsam mit einem pharmazeutischen Träger oder Excipienten enthält.

22. Pharmazeutische Zubereitungen nach Anspruch 21 zur Verwendung als geschwürhemmendes Mittel.

23. Pharmazeutische Zubereitungen nach Anspruch 21 zur Behandlung von an Störungen des Magen-Darmtraktes leidenden Patienten.

24. Zwischenverbindung der Formel V

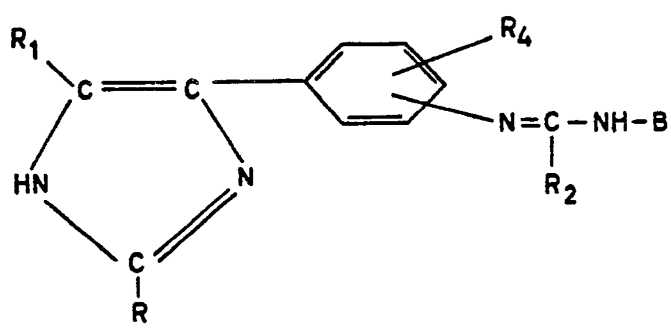

(V)

worin R, $R_1$, $R_2$ und $R_4$ wei in Anspruch 1 definiert sind und B wie in Anspruch 17 definiert ist, zur Durchführung des Verfahrens nach Anspruch 17.

**Patentansprüche für den Vertragsstaat: AT**

1. Verfahren zur Herstellung von

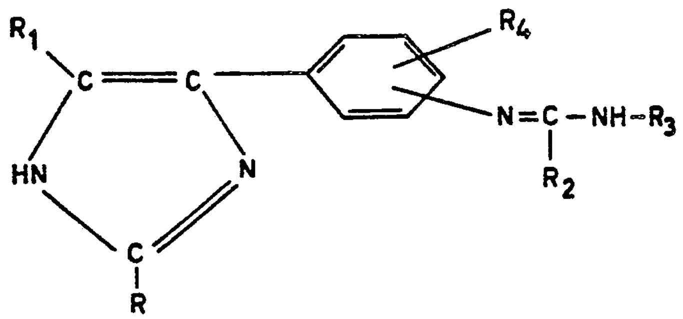

(I)

in welcher R eine geradkettige oder verzweigte $C_{1-4}$ Alkylgruppe, eine Hydroxygruppe, eine $C_{1-3}$ Alkoxygruppe, eine Mercapto-, eine $C_{1-3}$ Alkylthiogruppe, eine Halogenatom, eine $C_{1-3}$ Alkylsulfinyl- oder $C_{1-3}$ Alkylsulphonylgruppe, eine Sulfamoylgruppe, eine Aminogruppe, welche durch eine oder zwei Alkylgruppen mit ein bis drei Kohlenstoffatomen substituiert sein kann, eine $C_{1-4}$ Alkanoylaminogruppe oder Phenylgruppe bedeutet; $R_1$ and $R_2$, welche gleich oder voneinander verschieden sein können, jeweils ein Wasserstoffatom oder eine $C_{1-4}$ Alkylgruppe bedeuten; $R_3$ eine geradkettige oder verzweigte $C_{1-8}$ Alkyl-, Hydroxy-($C_{1-6}$)alkyl, ($C_{1-6}$) Alkoxy-($C_{1-6}$)alkyl, ($C_{1-6}$) Alkylthio-($C_{1-6}$)alkyl, Cyano ($C_{1-6}$)alkyl-, eine $C_{3-5}$ Alkenylgruppe, eine Alkynylgruppe mit 3 oder 4 Kohlenstoffatomen, eine Cyanogruppe, eine Arylgruppe, welche gegebenenfalls durch Halogen substituiert sein kann, eine Benzylgruppe, eine ($C_{3-6}$) Cycloalkylgruppe, eine ($C_{3-6}$) Cycloalkyl-($C_{1-2}$)alkylgruppe, eine aromatischer 5- bis 6-gliedriger heterocyclischer Ring, welcher ein Heteroatom enthält, bedeutet; $R_4$ ein Wasserstoffatom, eine $C_{1-3}$ Alkyl- oder $C_{1-3}$ Alkoxygruppe, ein Halogenatom, eine Cyano- oder Carbamoylgruppe bedeutet, tautomere Formen davon und Säureadditionssalze davon, welches die Umsetzung einer Verbindungen der Formel II

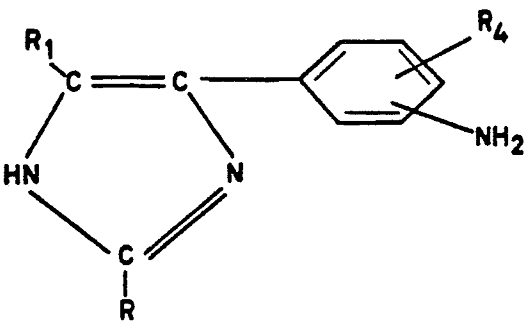

(II)

worin R, $R_1$ und $R_4$ wie oben definiert sind, mit einer Verbindung der Formel III

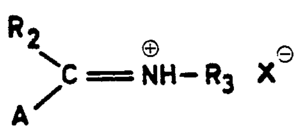

(III)

in welcher $R_2$ und $R_3$ wie oben definiert sind, $X^{\ominus}$ das Anion einer organischen Säure darstellt und A eine Benzyloxygruppe, Chlor oder eine Ethoxygruppe bedeutet, umfaßt.

2. Verfahren nach Anspruch 1, in welchem das Anion einer anorganischen Säure Hydrochlorid oder Fluorborat bedeutet.

3. Verfahren nach Anspruch 1, bei welchem die Verbindung der Formel (III) als Base reagiert.

4. Verfahren nach einem der Ansprüche 1 bis 3, bei welchem die Reaktion in Gegenwart eines inerten organischen Lösungsmittels durchgeführt wird.

5. Verfahren nach Anspruch 4, bei welchem das inerte organische Lösungsmittel Ethanol, Dioxan oder Aceton bedeutet.

6. Verfahren nach einem der Ansprüche 1 bis 5, bei welchem die Reaktion bei einer Temperatur von 20 bis 60°C durchgeführt wird.

7. Verfahren zur Herstellung von Verbindungen der allgemeinen Formel (I) nach Anspruch 1, welches die Umsetzung einer Verbindung der Formel V

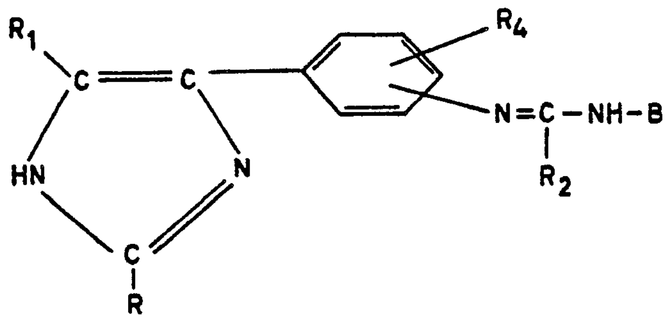

(V)

in welcher R, $R_1$, $R_2$ und $R_4$ wie in Anspruch 1 definiert sind, B eine geeignete Abgangsgruppe bedeutet, mit einem Amin der Formel VI

$$H_2N—R_3 \qquad (VI)$$

in welcher $R_3$ wie in Anspruch 1 definiert ist, umfaßt.

8. Verfahren nach Anspruch 7, bei welchem die geeignete Abgangsgruppe eine Cyano-, Acetyl-, Ethoxycarbonyl- oder Carbamylgruppe umfaßt.

9. Verfahren nach den Ansprüchen 7 bis 8, bei welchem die Reaktion in Gegenwart von Wasser durchgeführt wird.

10. Verfahren nach den Ansprüchen 7 bis 9, bei welchem die Reaktion bei Raumtemperatur durchgeführt wird.

11. Verfahren nach Anspruch 7, worin die Salze mit Chlorwasserstoff, Schwefel-, Malein- oder Fumarsäure gebildet werden

12. Verfahren nach Anspruch 1, worin die Verbindung der Formel I N-Ethyl-N'-[4-(2-methyl-imidazol-4-yl)phenyl]formamidin ist.

13. Verfahren nach Anspruch 1, worin die Verbindung der Formel I N-Isopropyl-N'-[4-(2-methyl-imidazol-4-yl)phenyl]formamidin ist.

14. Verfahren nach Anspruch 1, worin die Verbindung der Formel I N-Allyl-N'-[4-(2-methyl-imidazol-4-yl)phenyl]formamidin ist.

15. Verfahren nach Anspruch 1, worin die Verbindung der Formel I N-tert.-Butyl-N'-[4-(2-methyl-imidazol-4-yl)phenyl]formamidin ist.

16. Verfahren nach Anspruch 1, worin die Verbindung der Formel I N-Isopropyl-N'-[4-(2-hydroxy-imidazol-4-yl)phenyl]formamidin ist.

17. Verfahren nach Anspruch 1, worin die Säureadditionssalze Malein-, Chlorwasserstoff-, Fumar- oder Schwefelsäureadditionssalze sind.

18. Verfahren zur Herstellung des neuen Zwischenproduktes der Formel V

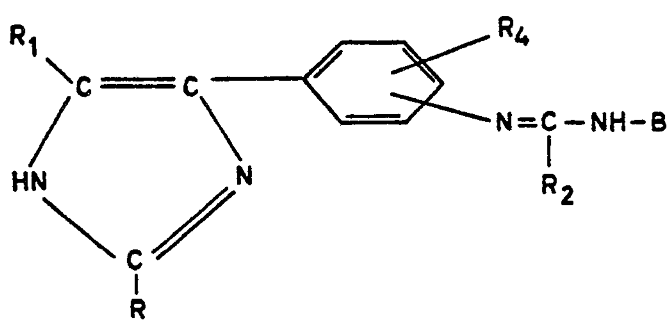

(V)

worin R, $R_1$, $R_2$ und $R_4$ wie in Anspruch 1 definiert sind und B wie in Anspruch 7 definiert ist, zur Durchführung des Verfahrens nach Anspruch 7.

**Revendications pour les Etats contractants: BE CH DE FR IT LI LU NL SE**

1. Composés de formule générale I

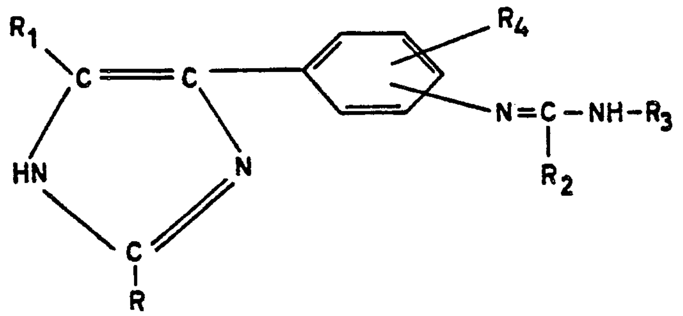

(I)

dans laquelle R représente un groupe alkyle linéaire ou ramifié $C_{1-4}$, un groupe hydroxy, un groupe alcoxy $C_{1-3}$, un radical mercapto, un groupe alkylthio $C_{1-3}$, un atome d'halogène, un groupe alkyl-sulfinyle $C_{1-3}$, ou alkylsulfonyle $C_{1-3}$, un groupe sulfamoyle, un groupe amino qui peut être substitué par un ou deux groupes alkyle ayant un à trois atomes de carbone, un groupe alkanoylamino $C_{1-4}$ ou phényle; $R_1$ et $R_2$ qui peuvent être les mêmes ou être différents représentent un atome d'hydrogène ou un groupe alkyle$C_{1-4}$; $R_3$ représente un groupe alkyle$C_{1-8}$ linéaire ou ramifié, alkyl($C_{1-6}$)-hydroxy, alkyl($C_{1-6}$)-alcoxy($C_{1-6}$), alkyl($C_{1-6}$)-alkylthio($C_{1-6}$), alkyl($C_{1-6}$)-cyano, un groupe alkényle$C_{3-5}$, un groupe alkynyle ayant trois ou quatre atomes de carbone, un groupe cyano, un groupe aryle éventuellement substitué par un halogène, un groupe benzyle, un groupe cycloalkyle ($C_{3-6}$), un groupe alkyl($C_{1-2}$)+cycloalkyle($C_{3-6}$), un anneau hétérocycle aromatique à 5 ou 6 membres contenant un hétéro-atome, $R_4$ représente un atome d'hydrogène, un groupe alkyle $C_{1-3}$ ou alcoxy $C_{1-3}$, un atome d'halogène, un groupe cyano ou carbamoyle, les formes tautomériques de ces corps et leurs sels d'addition acide.

2. Sels d'addition acide, compatibles physiologiquement des composés de formule (I) de la revendication 1.

3. Sels de la revendication 2 obtenus avec l'acide chlorhydrique, maléique ou fumarique.

4. N-Ethyl-N'-[4-(2-méthyl-imidazol-4-yl)phényl]formamidine.

5. N-Isopropyl-N'-[4-(2-méthyl-imidazol-4-yl)phényl]formamidine.

6. N-Allyl-N'-[4-(2-méthyl-imidazol-4-yl)phényl]formamidine.

7. N-t.Butyl-N'-[4-(2-méthyl-imidazol-4-yl)phényl]formamidine.

8. N-Isopropyl-N'-[4-(2-hydroxy-imidazol-4-yl)phényl]formamidine.

9. Sels d'addition acide compatibles physiologiquement des composés des revendications 4 à 8.

10. Sels d'addition avec l'acide maléique, chlorhydrique, fumarique ou sulfurique des composés des revendications 4 à 8.

11. Procédé pour la préparation de composés de formule générale (I) de la revendication 1 consistant à faire réagir un composé de formule II

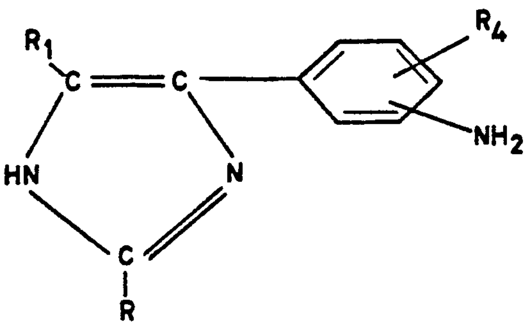

(II)

dans laquelle R, $R_1$ et $R_4$ sont tels que définis à la revendication 1, avec un composé de formule III,

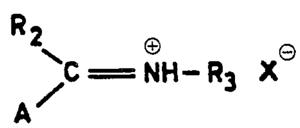

(III)

dans laquelle $R_2$ et $R_3$ sont tels que définis à la revendication 1, $X^{\ominus}$ représente l'anion d'un acide inorganique et A représente un groupe benzoyloxy, le chlore ou un groupe éthoxy.

12. Procédé selon la revendication 11 selon lequel l'anion d'un acide inorganique comprend un chlorhydrate ou un fluoborate.

13. Procédé selon la revendication 11 selon lequel un composé de formule (III) est mis à réagir comme base.

14. Procédé selon les revendications 11 à 13 selon laquelle la réaction est exécutée en présence d'un solvant organique inerte.

15. Procédé selon la revendication 14 selon lequel le solvant organique inerte comprend l'éthanol, le dioxane ou l'acetone.

16. Procédé selon les revendications 11 à 15 selon lequel la réaction est effectuée à une température de 20 à 60°C.

17. Procédé pour la préparation de composés de formule générale (I) de la revendication 1, comprenant la réaction d'un composé de formule V,

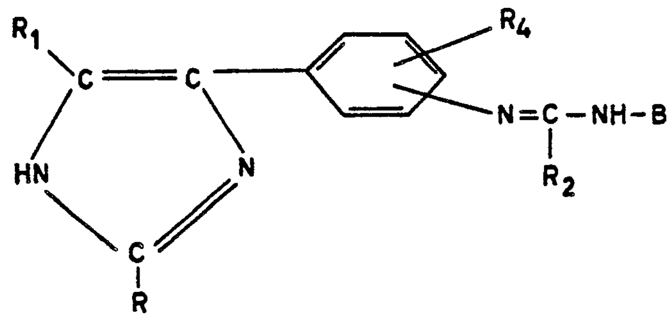

(V)

dans laquelle R, $R_1$, $R_2$ et $R_4$ sont tels que définis à la revendication 1, B représente un groupe convenable pouvant être éliminé, avec une amine de formule VI

$$H_2N—R_3 \qquad (VI)$$

dans laquelle $R_3$ est tel que défini à la revendication 1.

18. Procédé selon la revendication 17 selon lequel le groupe convenable pouvant être éliminé comprend un groupe cyano, un groupe acétyle un groupe étoxycarbonyle ou un groupe carbamyle.

19. Procédé selon les revendications 17 et 18 selon lequel la réaction est effectuée en présence d'eau.

20. Procédé selon les revendications 17 à 19 selon lequel la réaction est effectuée à la température de la pièce.

21. Compositions pharmaceutiques comprenant comme ingredient actif au moins un composé de formule générale (I) tel que défini à la revendication 1 ou un tautomère ou un sel d'addition acide physiologiquement compatible, en association avec un véhicule ou un excipient pharmaceutique.

22. Compositions pharmaceutiques de la revendication 21, destinées à être utilisées comme anti-ulcéreux.

23. Compositions pharmaceutiques de la revendication 21 pour le traitement de patients souffrant de désordres du tractus gastrointestinal.

24. Composé intermédiaire de formule V,

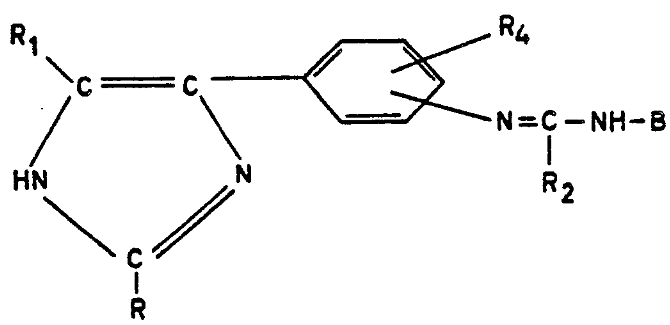

(V)

dans laquelle R, $R_1$, $R_2$ et $R_4$ sont tels que définis dans la revendication 1 et B est tel que défini dans la revendication 17 pour la mise en oeuvre du procédé de la revendication 17.

**Revendications pour l'Etat contractant: AT**

1. Procédé pour la préparation de

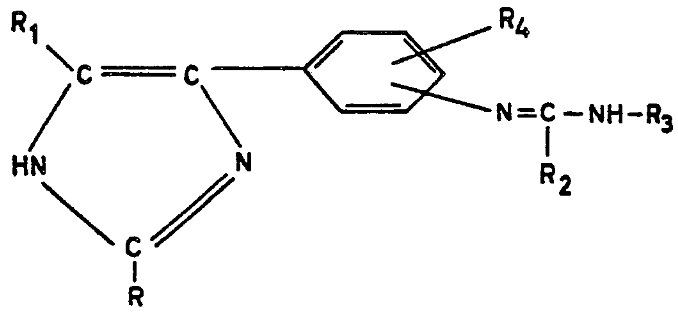

(I)

dans laquelle R représente un groupe alkyle linéaire ou ramifié $C_{1-4}$, un groupe hydroxy, un groupe alcoxy $C_{1-3}$, un radical mercapto, un groupe alkylthio $C_{1-3}$, un atome d'halogène, un groupe alkyl-sulfinyle $C_{1-3}$, ou alkylsulfonyle $C_{1-3}$, un groupe sulfamoyle, un groupe amino qui peut être substitué par un ou deux groupes alkyle ayant un à trois atomes de carbone, un groupe alkanoylamino $C_{1-4}$ ou phényle; $R_1$ et $R_2$ qui peuvent être les mêmes ou être différents représentent un atome d'hydrogène ou un groupe alkyle$C_{1-4}$; $R_3$ représente un groupe alkyle$C_{1-8}$ linéaire ou ramifié, alkyl($C_{1-6}$)-hydroxy, alkyl($C_{1-6}$)-alcoxy($C_{1-6}$), alkyl($C_{1-6}$)-alkylthio($C_{1-6}$), alkyl($C_{1-6}$)-cyano, un groupe alkényle$C_{3-5}$, un groupe alkynyle ayant trois ou quatre atomes de carbone, un groupe cyano, un groupe aryle éventuellement substitué par un halogène, un groupe benzyle, un groupe cycloalkyle ($C_{3-6}$), un groupe alkyl($C_{1-2}$)+cycloalkyle($C_{3-6}$), un anneau hétérocycle aromatique à 5 ou 6 membres contenant un hétéro-atome, $R_4$ représente un atome d'hydrogène, un groupe alkyle $C_{1-3}$ ou alcoxy $C_{1-3}$, un atome d'halogène, un groupe cyano ou carbamoyle, les formes tautomériques de ces corps et leurs sels d'addition acide, et leurs sels d'addition acide compatibles physiologiquement, qui consiste à faire réagir un composé de formule II

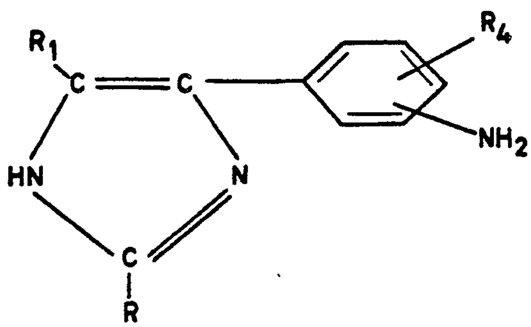

(II)

dans laquelle R, $R_1$, et $R_4$ sont tels que définis à la revendication 1, avec un composé de formule III,

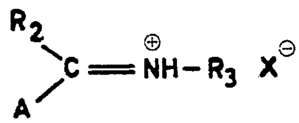

(III)

dans laquelle $R_2$ et $R_3$ sont tels que définis à la revendication 1, $X^{\ominus}$ représente l'anion d'un acide inorganique et A représente un groupe benzoyloxy, le chlore ou un groupe éthoxy.

2. Procédé selon la revendication 1 selon lequel l'anion d'un acide inorganique comprend un chlorhydrate ou un fluoborate.

3. Procédé selon la revendication 1 selon lequel un composé de formule (III) est mis à réagir comme base.

4. Procédé selon les revendication 1 à 3 selon lequel la réaction est exécutée en présence d'un solvant organique inerte.

5. Procédé selon la revendication 4 selon lequel le solvant organique inerte comprend l'éthanol, le dioxane ou l'acétone.

6. Procédé selon les revendications 1 à 5 selon lequel la réaction est effectuée à une température de 20 à 60°C.

7. Procédé pour la préparation de composés de formule générale (I) tels que spécifiés à la revendication 1, comprenant la réaction d'un composé de formule V,

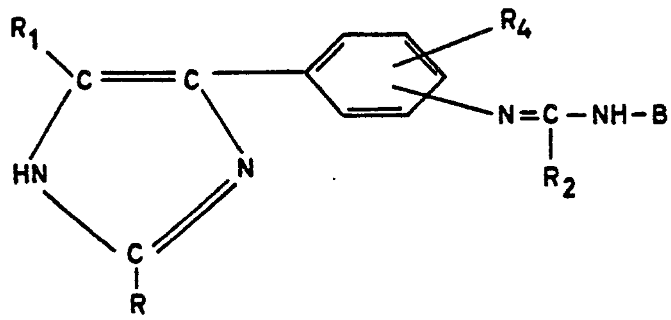

(V)

dans laquelle R, $R_1$, $R_2$ et $R_4$ sont tels que définis à la revendication 1, B représente un groupe convenable pouvant être éliminé, avec une amine de formule VI

$$H_2N—R_3 \qquad (VI)$$

dns laquelle $R_3$ est tel que défini à la revendication 1.

8. Procédé selon la revendication 7 selon lequel le groupe convenable pouvant être éliminé comprend un groupe cyano, un groupe acétyle un groupe étoxycarbonyle ou un groupe carbamyle.

9. Procédé selon les revendications 7 et 8 selon lequel la réaction est effectuée en présence d'eau.

10. Procédé selon les revendications 7 à 9 selon lequel la réaction est effectuée à la température de la pièce.

11. Procédé selon la revendication 7 selon lequel lesdits sels sont obtenus avec l'acide chlorhydrique, sulfurique, maléique ou fumarique.

12. Procédé selon la revendication 1, avec lequel ledit composé de formule (I) est N-Ethyl-N'-[4-(2-méthyl-imidazol-4-yl)phényl]formamidine.

13. Procédé selon la revendication 1, avec lequel ledit composé de formule (I) est N-Isopropyl-N'-[4-(2-méthyl-imidazol-4-yl)phényl]formamidine.

14. Procédé selon la revendication 1, avec lequel ledit composé de formule (I) est N-Allyl-N'-[4-(2-méthyl-imidazol-4-yl)phényl]formamidine.

15. Procédé selon la revendication 1, avec lequel ledit composé de formule (I) est N-t.Butyl-N'-[4-(2-méthyl-imidazol-4-yl)phényl]formamidine.

16. Procédé selon la revendication 1, avec lequel ledit composé de formule (I) est N-Isopropyl-N'-[4-(2-hydroxy-imidazol-4-yl)phényl]formamidine.

17. Procédé selon la revendication 1, avec lequel lesdits sels d'addition acide sont les sels d'addition avec l'acide maléique, chlorhydrique, fumarique ou sulfurique.

18. Procédé pour la préparation d'un nouveau composé intermédiaire de formule V

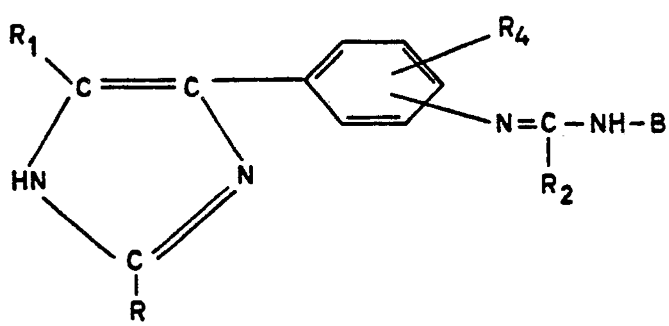

(V)

dans laquelle R, $R_1$, $R_2$, et $R_4$ sont tels que définis à la revendication 1 et B est telle que définie à la revendication 7 pour la mise en oeuvre du procédé selon la revendication 7.